# EUROPÄISCHE PATENTSCHRIFT

(11) **EP 0 799 817 B1**
(45) Veröffentlichungstag und Bekanntmachung des Hinweises auf die Patenterteilung: **23.06.1999**
(21) Anmeldenummer: 97104835.0
(22) Anmeldetag: 21.03.1997
(51) Int. Cl.: C07C 209/72, C07C 211/35, C07C 211/36

(54) **Verfahren zur Herstellung eines Gemisches von Amino-methyl-cyclohexanen und Diamino-methyl-cyclohexanen**
Process for the preparation of a mixture of amino-methyl-cyclohexane and diamino-methyl-cyclohexane
Procédé pour la préparation d'un mélange d'amino-méthyl-cyclohexane et de diamino-méthyl-cyclohexane

(30) Priorität: 03.04.1996 DE 19613332
(43) Veröffentlichungstag der Anmeldung: 08.10.1997
(73) Patentinhaber: BAYER AG, 51368 Leverkusen (DE)
(72) Erfinder: Darsow, Gerhard, Dr., 47804 Krefeld (DE); Petruck, Gerd-Michael, Dr., 40699 Erkrath (DE)

(56) Entgegenhaltungen:
- EP-A- 0 091 028
- EP-A- 0 501 265
- DE-A- 2 024 858
- DE-A- 2 506 348

## Beschreibung

Die Erfindung betrifft ein kontinuierliches Verfahren zur Herstellung eines Gemisches aus Amino-methyl-cyclohexanen und Diamino-methyl-cyclohexanen in variablen Mengen durch katalytische Druckhydrierung von Diamino-toluolen mit Wasserstoff bei erhöhter Temperatur unter Einsatz von durch Reduktion von Formkörpern aus verpreßten Pulvern von Kobalt-, Mangan-, Kupfer- und Erdalkalimetall(hydr)oxiden hergestellten Katalysatoren, wobei die Formkörper einen Gehalt an einem oder mehreren (Hydr)Oxiden von Metallen der V. und/oder VI. Nebengruppe des Periodensystems der Elemente (Mendelejew) aufweisen können.

Amino-methyl-cyclohexane finden Verwendung zur Herstellung von Alterungsschutzmitteln für Kautschuke und Kunststoffe, als Korrosionsschutzmittel sowie als Vorprodukte für Textilhilfsmittel und Pflanzenschutzmittel.

Diamino-methyl-cyclohexane finden Verwendung zur Herstellung von Pulverlackhärtern, Epoxidhärtern, lichtechten Lackharzen sowie wäßrigen Lackharzdispersionen.

Es ist bekannt, Diamino-methyl-cyclohexane durch Druckhydrierung von Diamino-toluolen herzustellen. Für diese Hydrierung werden als Katalysatoren Nickel-Legierungen mit Molybdän, Ruthenium, Tantal, Titan (EP 091 028) oder Kobaltoxid (DE 16 18 638/ DE 2 024 858 / JP 48/32 845 (1973)) eingesetzt, wobei in den Versuchsbeispielen ausschließlich diskontinuierliche Verfahren beschrieben sind und als Reaktionsprodukte ausschließlich Diamino-methyl-cyclohexane genannt werden.

Andere Verfahren in Batch-Fahrweise verwenden hauptsächlich kostspielige Edelmetalle auf Trägern, wie Rhodium auf Al₂O₃ (JP 59/216 852 (1984)), Platin und Palladium auf Kohle (US 3 520 928) oder Ruthenium auf Al₂O₃, SiO₂ bzw. Kohle (DE 2 132 547 / JP 51/68 539 (1976)) oder Ruthenium, Chrom und Mangan auf Al₂O₃ (DE-OS 2 502 893 / DE-OS 2 745 172).

Monoamino-methyl-cyclohexane entstehen bei diesen Reaktionen offenbar überhaupt nicht. Um Amino-methyl-cyclohexane in größeren Mengen zu erhalten, werden sie nach separaten Verfahren hergestellt. So wird z.B. 1-Amino-4-methyl-cyclohexan durch Hydrierung von p-Toluidin an einem Palladium (Platin)/Kohle-Katalysator hergestellt (US 3 520 928). Aus DE-A-2506348 ist ein Katalysator bekannt, der Kobalt , Mangan, Kupfer und Alkali enthält und zur katalytischen Hydrierung von Diphenylamin verwendet wird.

Ein gemeinsames Problem der Verfahren zur Kernhydrierung methyl-substituierter aromatischer Amine besteht in der z.T. beträchtlichen Bildung von methyl-und amino-substituierten Dicyclohexyl-aminen als unbrauchbaren Nebenprodukten. Es besteht daher der Wunsch, ein kontinuierliches auch im technischen Maßstab brauchbares Festbettverfahren zu entwickeln, nach welchem sowohl Monoamino-methyl-cyclohexane als auch Diamino-methyl-cyclohexane in einem gewünschten Mengenverhältnis hergestellt werden können, bei welchem Verluste durch die unerwünschte Bildung von methyl- und amino-substituierten Dicyclohexyl-aminen vermieden und bei welchem weiterhin eine möglichst hohe Lebensdauer des verwendeten Katalysators angestrebt wird.

Überraschenderweise wurde jetzt gefunden, daß die obengenannten Anforderungen durch den Einsatz preiswerter (hydr)oxidischer Festbettkatalysatoren erfüllt werden, die frei von inaktivem Trägermaterial sind und daher nach Gebrauch einfach aufgearbeitet und entsorgt werden können.

Die Erfindung betrifft somit ein kontinuierliches Verfahren zur Herstellung eines Gemisches aus Amino-methyl-cyclohexanen und Diamino-methyl-cyclohexanen der Formeln 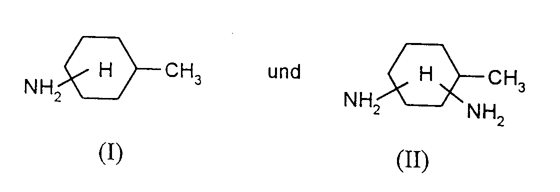 durch katalytische Hydrierung von Diamino-toluolen der Formel 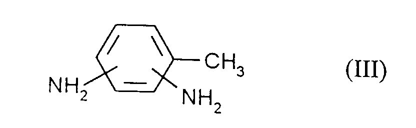 mit Wasserstoff bei Reaktionstemperaturen von 150 bis 260°C und einem H₂-Druck von 20 bis 500 bar, das dadurch gekennzeichnet ist, daß als Katalysatoren dienende trägerfreie, reduzierte Formkörper aus verpreßten Pulvern von Kobalt-, Mangan-, Kupfer- und Erdalkalimetall(hydr)oxiden, wobei die Formkörper einen Gehalt an einem oder mehreren (Hydr)Oxiden von Metallen der V. und/oder VI. Nebengruppe des Periodensystems der Elemente (Mendelejew) aufweisen können, eingesetzt werden.

Für die erfindungsgemäß zu verwendenden Katalysatoren betragen die Co-Anteile 40 bis 65 Gew.-%, die Mn-Anteile 10 bis 20 Gew.-%, die Cu-Anteile 0,1 bis 0,5 Gew.-%, die Erdalkalimetall-Anteile 0,2 bis 5 Gew.-% und die gegebenenfalls vorliegenden Anteile an Metallen der V. und/oder VI. Nebengruppe des Periodensystems insgesamt 0,2 bis 5 Gew.-%, jeweils gerechnet als Metall. Der Rest zu 100 Gew.-% ist Sauerstoff für die in oxidischer Form vorliegenden Metalle.

Von den Erdalkalimetallen eignen sich beispielsweise Magnesium, Calcium, Strontium und Barium, vorzugsweise Strontium und Barium. Von den Metallen der V. Nebengruppe eignen sich beispielsweise Vanadium, Niob und Tantal, von den Metallen der VI. Nebengruppe beispielsweise Chrom, Molybdän und Wolfram. Die Metalle der V. und/oder VI. Nebengruppe können ebenso wie die Erdalkalimetalle entweder einzeln oder als Mischung mehrerer dieser Elemente zum Einsatz kommen.

Die Herstellung der trägerfreien Formkörper kann nach gebräuchlichen Methoden durch Verpressen der Metall(hydr)oxid-Pulvermischungen (gegebenenfalls nach vorausgegangener Temperierung bei höheren Temperaturen), beispielsweise auf Tablettier- oder Pelletier-Maschinen, unter hohem Druck erfolgen, wobei zur Verbesserung des Haftvermögens der Metalloxidpartikel auch Graphit und/oder Klebstoffe in Mengen von 0,5 bis 3 Gew.-%, bezogen auf das Gesamtgewicht der zu verpressenden Bestandteile, zum Einsatz kommen können. Beispiele für Formkörper sind Tabletten, Kugeln oder Granulate mit Durchmessern von 3 bis 7 mm. Tablettierte Formkörper können weiterhin zur Vergrößerung der äußeren Oberfläche mit einer axialen Durchbohrung versehen sein. Solche Formkörper haben makroskopisch betrachtet eine glatte Oberfläche. Die verpreßten Metalloxid-Formkörper haben eine hohe Druckfestigkeit von 300 bis 800 N/cm², bevorzugt 400 bis 600 N/cm², auf die ebene Formkörperoberfläche bei Benutzung eines flächigen Druckstempels bzw. von 50 bis 200 N, bevorzugt 80 bis 140 N, auf die gewölbte Formkörperoberfläche bei Benutzung eines messerförmigen Druckgebers. Die innere Oberfläche der verpreßten Metalloxidpulver beträgt 30 bis 200 m²/g, bevorzugt 80 bis 160 m²/g. Die Druckfestigkeit der trägerfreien Formkörper kann nach DIN 50 106, die innere Oberfläche nach F.M. Nelsen und F.T. Eggertsen, Analyt. Chem. 30 (1958), 1387-1392 bzw. S.J. Gregg und S.W. Sing, Adsorption, Surface Area and Porosity, London 1982, Kap. 2 und 6 bestimmt werden.

Vor ihrem Einsatz werden die Formkörper aus verpreßten Oxidpulvern reduziert. Dies geschieht vorzugsweise durch den Einsatz eines Reduktionsgases, das aus einer Inertgas/Wasserstoff-Mischung besteht, in der der Wasserstoffgehalt zu Beginn bei 10 bis 15 Vol.-% liegt. Als Inertgas wird bevorzugt Stickstoff eingesetzt. Die Reduktion erfolgt beispielsweise innerhalb eines Zeitraumes von etwa 24 Stunden bei einer Reduktionstemperatur von 160 bis 280°C und bei 20 bis 300 bar Druck, wobei in der Endphase der Reduktion der Stickstoffanteil der Gasmischung mehr und mehr vermindert wird, bis diese schließlich aus reinem Wasserstoff besteht. Die Reduktion ist beendet, wenn kein Wasserstoff mehr verbraucht und infolgedessen kein Reaktionswasser mehr gebildet wird. Die Reduktion findet bevorzugt nach Anordnung der zu reduzierenden Formkörper im Hydrierreaktor statt.

Als Ausgangsstoffe für die Hydrierung nach dem erfindungsgemäßen Verfahren kommen beispielsweise in Betracht: 2,4-Diamino-toluol, 2,5-Diamino-toluol, 2,6-Diamino-toluol oder Mischungen aus diesen Verbindungen.

Das erfindungsgemäße Verfahren kann mit oder ohne Lösungsmittel durchgeführt werden. Geeignete, unter den Reaktionsbedingungen inerte Lösungsmittel sind beispielsweise Methanol, Ethanol, Isopropanol.

Mit Hilfe des Einsatzes der beschriebenen Katalysatoren entstehen in dem erfindungsgemäßen Verfahren Gemische aus Amino-methyl-cyclohexanen und Diamino-methyl-cyclohexanen, wobei überraschend in Abhängigkeit von der Hydriertemperatur das Mengenverhältnis der Amine so verändert werden kann, daß mit steigender Temperatur mehr Amino-methyl-cyclohexane gebildet werden und mit sinkender Temperatur der umgekehrte Effekt erreicht wird.

Das erfindungsgemäße Verfahren wird beispielsweise mit dem im Festbett angeordneten Katalysator in der Gas- bzw. Rieselphase durchgeführt. Es wird bei Wasserstoffüberschuß gearbeitet; die H₂-Menge beträgt das 10- bis 120-fache der Menge, bevorzugt die 10- bis 80-fache Menge, die für die Hydrierung eines Benzolkerns erforderlich ist.

Es wird bei 150 bis 260°C, bevorzugt bei 160 bis 250°C und bei einem Druck von mindestens 20 bar, bevorzugt mindestens 100 bar, besonders bevorzugt mindestens 200 bar gearbeitet. Die Obergrenze des zur Anwendung gelangenden Drucks ergibt sich sowohl aus technischen als auch aus wirtschaftlichen Überlegungen und liegt bei 500 bar, bevorzugt bei 200 bis 400 bar.

Als Katalysatorbelastung wird eine Menge von 0,05 bis 2 kg, bevorzugt 0,1 bis 1 kg, besonders bevorzugt 0,15 bis 0,6 kg Diamino-toluole pro Liter Katalysator pro Stunde eingestellt. Eine geringe Veränderung des erzielten Anteils an Amino-methyl-cyclohexanen durch veränderte Aktivität des Katalysators im Laufe besonders langer Reaktionsperioden kann durch ein geringes Nachstellen der Reaktionstemperatur oder der anderen Parameter ausgeglichen werden. Diese Verhältnisse können durch die Analytik des Reaktionsgemisches verfolgt werden.

Die Anordnung des erfindungsgemäß einzusetzenden Katalysators kann in verschiedenen, dem Fachmann für solche Zwecke grundsätzlich bekannten Apparaten erfolgen. In vorteilhafter Weise wird das erfindungsgemäße Verfahren in Röhrenreaktoren mit einer oder mehreren Röhren durchgeführt. Die Reaktionsrohre können Längen von beispielsweise 2 bis 20 m und innere Durchmesser von 20 bis 800 mm besitzen. Die Katalysatoren haben beispielsweise Abmessungen von 2 bis 10 mm und liegen beispielsweise als Strangpreßlinge, Pillen oder Kugeln vor. Bei größeren Querschnitten der Reaktoren können die Katalysator-Formkörper auch auf Horden, in Drahtkörben oder in ähnlich vorgeordneter Form angeordnet werden.

Die erfindungsgemäß eingesetzten Katalysatoren zeigen sehr hohe Standzeiten; bisher sind 12.000 bis 15.000 Stunden beobachtet worden, nach denen die Versuche ohne erkennbares Nachlassen der Aktivität abgebrochen wurden.

Die nach der Hydrierung erhaltenen Reaktionsgemische enthalten praktisch keine methyl-substituierten Amino-di-N-cyclohexane, so daß besonders hohe Gehalte an Amino-methyl-cyclohexanen und Diamino-methyl-cyclohexanen erzielt werden können.

Die Hydriergemische können durch einfache Destillation aufgearbeitet werden. Für eine solche Aufarbeitung kann es vorteilhaft sein, die jeweiligen Diamino-toluole nicht vollständig umzusetzen, weil bei der Bildung der Monoamino-methyl-cyclohexane freiwerdendes Ammoniak sehr gut in den Diamino-toluolen löslich ist und so aus dem Reaktionsabgas entfernt werden kann. Bei der Destillation der Reaktionsprodukte wird das gelöste Ammoniak als erstes abdestilliert, kondensiert und damit für eine Weiterverwendung nutzbar; nicht vollständig umgesetzte Diamino-toluole können wieder in die Reaktion zurückgeführt werden. Auch der nicht verbrauchte Anteil des im Überschuß zugesetzten Wasserstoffs kann in die Reaktion zurückgeführt werden, wobei in vorteilhafter Weise der größte Teil dieses nicht umgesetzten Wasserstoffs in einem Hochdruckabscheider zurückgewonnen wird, so daß die Kompressionsarbeit für den Wasserstoff nicht noch einmal geleistet zu werden braucht.

Die erfindungsgemäß hergestellten Amino-methyl-cyclohexane und Diamino-methyl-cyclohexane werden nach erfolgreicher destillativer Trennung in einer Reinheit von mindestens 99,9 Gew.-% erhalten. In dieser Reinheit sind die genannten Verbindungen in der Regel für alle weiterverarbeitenden Prozesse einsetzbar.

Die Variabilität des erfindungsgemäßen Verfahrens zeigt sich in einer starken Zunahme des Anteils an Amino-methyl-cyclohexanen gegenüber den Diamino-methyl-cyclohexanen mit steigender Temperatur und sonst gleichen Bedingungen. So erhält man beispielsweise eine Steigerung des Anteils an Amino-methyl-cyclohexanen im Temperaturbereich von etwa 200 bis 230°C auf das 2- bis 10-fache des Anteils im Temperaturbereich von 170 bis 185°C.

### Beispiele

### Beispiel 1

Ein senkrecht stehendes, wärmeisoliertes Hochdruckrohr aus nichtrostendem Stahl von 30 mm Innendurchmesser und 1 m Länge, das vorher mit Stickstoff sauerstofffrei gespült worden war, wurde mit 400 ml eines durch Tablettierung von Pulvern von Co-, Mn-, Cu-, Ba- und Mo-Oxiden hergestellten Hydrierungskatalysatoren gefüllt. Der Co-Gehalt der Tabletten lag bei 53 Gew.-%, der Mn-Gehalt bei 14 Gew.-%, der Cu-Gehalt bei 0,18 Gew.-%, der Ba-Gehalt bei 1,1 Gew.-% und der Mo-Gehalt bei 1,2 Gew.-% (Rest zu 100 %: Sauerstoff). Die Tabletten hatten bei einer Zylinderhöhe von 5 mm und einem Durchmesser von 5 mm eine Druckfestigkeit von 420 N/cm² auf die ebene Querschnittsfläche und von 125 N auf die gewölbte Mantelfläche sowie eine innere Oberfläche von 168 m²/g. Zur Aktivierung des Katalysators wurden die Tabletten zunächst 6 Stunden im Stickstoffstrom nachgetrocknet (Temperatur: max. 200°C, Menge 5 Nm³ N₂/h). Die Aktivierung erfolgte sodann unter einem Stickstoffdruck von 200 bar bei einer Temperatur zwischen 180 und 260°C, wobei dem Stickstoff allmählich Wasserstoff zugemischt wurde, dessen Zumischungsanteil anfangs 10 bis 15 Vol.-% betrug. Innerhalb von 24 Stunden wurde der Stickstoffanteil mehr und mehr vermindert, bis schließlich nur Wasserstoff den Reaktor durchströmte. Die Aktivierung war beendet, sobald sich im nachgeschalteten Abscheider kein Reaktionswasser mehr ansammelte.

Nach der Aktivierung des Hydrierkatalysators wurde der Wasserstoffdruck im Reaktorsystem auf 300 bar erhöht. Anschließend wurden stündlich in der Rieselphase 160 g 2,4-Diamino-toluol gemeinsam mit 1500 Nl Wasserstoff unter einem Druck von 300 bar von oben nach unten absteigend durch das Hochdruckrohr gepumpt, wobei das 2,4-Diamino-toluol vor Eintritt in den Reaktor in einem vorgeschalteten, elektrisch beheizten Wärmeaustauscher auf eine Temperatur von 180°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur von ≤60°C abgekühlt und in einem Gasabscheider von überschüssigem Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angaben in Flächen-%; der Rest zu 100 % sind Nebenprodukte):

| Laufzeit (h) | Temperatur (°C) | 2- und 4-Amino-methyl-cyclohexan (F-%) | 2,4-Diamino-methyl-cyclohexan (F-%) | 2,4-Diamino-toluol (F-%) |
|---|---|---|---|---|
| 254 | 250 | 36,8 | 53,9 | 2,0 |
| 278 | 245 | 26,0 | 63,0 | 6,6 |
| 302 | 240 | 25,2 | 48,9 | 22,2 |
| 326 | 235 | 23,2 | 37,2 | 34,6 |
| 350 | 220 | 18,8 | 28,8 | 35,1 |
| 422 | 210 | 15,5 | 24,1 | 58,0 |
| 494 | 200 | 8,3 | 15,0 | 75,8 |
| 518 | 180 | 3,9 | 9,1 | 86,5 |

### Beispiel 2

Ein senkrecht stehendes, wärmeisoliertes Hochdruckrohr aus nichtrostendem Stahl von 45 mm Innendurchmesser und 1 m Länge, das vorher mit Stickstoff sauerstofffrei gespült worden war, wurde mit 1.400 ml eines durch Tablettierung von Pulvern von Co-, Mn-, Cu-, Ba- und Mo-Oxiden hergestellten Hydrierungskatalysators gefüllt. Der Co-Gehalt der Tabletten lag bei 53 Gew.-%, der Mn-Gehalt bei 14 Gew.-%, der Cu-Gehalt bei 0,2 Gew.-%, der Ba-Gehalt bei 0,9 Gew.-% und der Mo-Gehalt bei 1,1 Gew.-%. Die Tabletten hatten bei einer Zylinderhöhe von 5 mm und einem Durchmesser von 5 mm eine Druckfestigkeit von 425 N/cm² auf die Querschnittsfläche und von 160 N auf die Zylindermantelfläche sowie eine innere Oberfläche von 180 m²/g.

Nach der Aktivierung des Hydrierkatalysators wie in Beispiel 1 wurde der Wasserstoffdruck im Reaktorsystem auf 300 bar erhöht. Anschließend wurden stündlich in der Rieselphase 240 g eines Gemisches aus 80 Gew.-% 2,4-Diamino-toluol und 20 Gew.-% 2,6-Diamino-toluol gemeinsam mit 5.000 Nl Wasserstoff unter einem Druck von 300 bar von oben nach unten absteigend durch das Hochdruckrohr gepumpt, wobei das Diamino-toluol-Gemisch vor Eintritt in den Reaktor in einem vorgeschalteten elektrisch beheizten Wärmeaustauscher auf eine Temperatur von 180°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur von <60°C abgekühlt und in einem Gasabscheider von überschüssigem Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angabe in Flächen-%; der Rest zu 100 % sind Nebenprodukte):

| Laufzeit (h) | Temperatur (°C) | 2- u. 4-Amino-methylcyclohexan (F-%) | 2,4- und 2,6-Diamino-methyl-cyclohexan (F-%) | 2,4- und 2,6-Diamino-toluol (F-%) |
|---|---|---|---|---|
| 54 | 180 | 4,8 | 25,9 | 66,8 |
| 102 | 200 | 5,3 | 36,5 | 58,7 |
| 324 | 210 | 10,3 | 65,4 | 20,3 |
| 396 | 215 | 22,2 | 59,2 | 18,6 |
| 588 | 230 | 38,0 | 50,5 | 6,1 |

### Beispiel 3

Ein senkrecht stehendes, wärmeisoliertes Hochdruckrohr aus nichtrostendem Stahl von 30 mm Innendurchmesser und 1 m Länge, das vorher mit Stickstoff sauerstofffrei gespült worden war, wurde mit 400 ml eines durch Tablettierung von Pulvern von Co-, Mn-, Cu-, Ba- und V-Oxiden hergestellten Hydrierungskatalysators gefüllt. Der Co-Gehalt der Tabletten lag bei 52 Gew.-%, der Mn-Gehalt bei 16 Gew.-%, der Cu-Gehalt bei 0,18 Gew.-%, der Ba-Gehalt bei 0,91 Gew.-% und der V-Gehalt bei 1,1 Gew.-% (Rest zu 100 Gew.-%: Sauerstoff). Die Tabletten hatten bei einer Zylinderhöhe von 5 mm und einem Durchmesser von 5 mm eine Druckfestigkeit von 420 N/cm² auf die Querschnittsfläche und von 160 N auf die Zylindermantelfläche sowie eine innere Oberfläche von 180 m²/g.

Die Tabletten wurden zunächst 6 Stunden im Stickstoffstrom getrocknet (Temperatur: max. 200°C, Menge: 5 Nm³ N₂/h). Die Aktivierung erfolgte unter einem Stickstoffdruck von 200 bar bei einer Temperatur zwischen 180 und 280°C, wobei dem Stickstoff allmählich Wasserstoff zugemischt wurde, dessen Zumischungsanteil anfangs 10 bis 15 Vol.-% betrug. Innerhalb von 24 Stunden wurde der Stickstoffanteil mehr und mehr vermindert, bis schließlich nur Wasserstoff den Reaktor durchströmte. Die Aktivierung war beendet, sobald sich im nachgeschalteten Abscheider kein Reaktionswasser mehr ansammelte.

Nach der Aktivierung des Hydrierkatalysators wurde der Wasserstoffdruck im Reaktorsystem auf 280 bar erhöht. Anschließend wurden stündlich in der Rieselphase 100 g eines Gemisches aus 80 Gew.-% 2,4-Diamino-toluol und 20 Gew.-% 2,6-Diamino-toluol gemeinsam mit 1.500 Nl Wasserstoff unter einem Druck von 280 bar von oben nach unten absteigend durch das Hochdruckrohr gepumpt, wobei das Diamino-toluol-Gemisch vor Eintritt in den Reaktor in einem vorgeschalteten elektrisch beheizten Wärmeaustauscher auf eine Temperatur von 160°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur von <60°C abgekühlt und in einem Gasabscheider von überschüssigem Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angabe in Flächen-%; der Rest zu 100 % sind Nebenprodukte):

| Laufzeit (h) | Temperatur (°C) | 2- und 4-Amino-methyl-cyclohexan (F-%) | 2,4- und 2,6-Diamino-methyl-cyclohexan (F-%) | 2,4- und 2,6-Diamino-toluol (F-%) |
|---|---|---|---|---|
| 80 | 180 | 4,6 | 43,8 | 49,5 |
| 124 | 200 | 6,8 | 46,2 | 43,9 |
| 198 | 210 | 12,4 | 51,5 | 33,8 |
| 240 | 215 | 24,8 | 59,1 | 15,2 |
| 588 | 230 | 31,7 | 61,2 | 5,4 |

### Beispiel 4

Ein senkrecht stehendes, wärmeisoliertes Hochdruckrohr aus nichtrostendem Stahl von 30 mm Innendurchmesser und 1 m Länge, das vorher mit Stickstoff sauerstofffrei gespült worden war, wurde mit 400 ml eines durch Tablettierung von Pulvern von Co-, Mn-, Cu- und Ba-Oxiden hergestellten Hydrierkatalysators gefüllt. Der Co-Gehalt der Tabletten lag bei 54 Gew.-%, der Mn-Gehalt bei 14,5 Gew.-%, der Cu-Gehalt bei 0,18 Gew.-% und der Ba-Gehalt bei 1,05 Gew.-%. Die Tabletten hatten bei einer Zylinderhöhe von 5 mm und einem Durchmesser von 5 mm eine Druckfestigkeit von 438 N/cm² auf die ebene Zylinderoberfläche und von 115 N auf die gewölbte Formkörperoberfläche sowie eine innere Oberfläche von 178 m²/g.

Die Tabletten wurden, wie in Beispiel 1 beschrieben, getrocknet und aktiviert. Nach der Aktivierung wurde der Wasserstoffdruck im Reaktorsystem auf 280 bar erhöht. Anschließend wurden stündlich in der Rieselphase 100 g eines Gemisches aus 80 Gew.-% 2,4-Diamino-toluol und 20 Gew.-% 2,6-Diamino-toluol gemeinsam mit 1,5 Nm³ Wasserstoff unter einem Druck von 280 bar durch das Hochdruckrohr gepumpt, wobei das zu hydrierende Diamino-toluol-Gemisch vor Eintritt in das Hochdruckrohr in einem vorgeschalteten elektrisch beheizten Wärmeaustauscher auf eine Temperatur von 175°C erhitzt wurde. Das das Reaktionsrohr verlassende Reaktionsprodukt wurde, wie in Beispiel 1 beschrieben, abgekühlt und vom überschüssigen Wasserstoff getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (der Rest zu 100 % sind Nebenprodukte):

| Laufzeit (h) | Tempera tur (°C) | 2- und 4-Aminomethyl-cyclohexan (F-%) | 2,4- und 2,6-Diamino-methylcyclohexan (F-%) | 2,4- und 2,6-Diamino-toluol (F-%) |
|---|---|---|---|---|
| 286 | 180 | 2,1 | 28,6 | 65,1 |
| 348 | 200 | 6,2 | 38,4 | 51,6 |
| 412 | 210 | 11,0 | 45,2 | 46,2 |
| 486 | 220 | 15,4 | 52,2 | 32,0 |
| 526 | 230 | 22,8 | 48,7 | 26,6 |
| 612 | 240 | 28,1 | 64,8 | 4,2 |
| 718 | 250 | 32,0 | 65,3 | 0,9 |

## Patentansprüche

1. Kontinuierliches Verfahren zur Herstellung eines Gemisches aus Amino-methyl-cyclohexanen und Diamino-methyl-cyclohexanen der Formeln 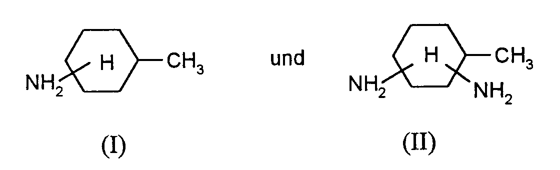 durch katalytische Hydrierung von Diamino-toluolen der Formel 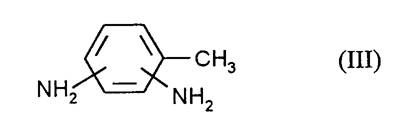 mit Wasserstoff bei Reaktionstemperaturen von 150 bis 260°C und einem H₂-Druck von 20 bis 500 bar, dadurch gekennzeichnet, daß als Katalysatoren dienende trägerfreie, reduzierte Formkörper aus verpreßten Pulvern von Kobalt-, Mangan-, Kupfer- und Erdalkalimetall(hydr)oxiden, wobei die Formkörper einen Gehalt an einem oder mehreren (Hydr)Oxiden von Metallen der V. und/oder VI. Nebengruppe des Periodensystems der Elemente (Mendelejew) aufweisen können, eingesetzt werden.

2. Verfahren nach Anspruch 1, wonach die zu reduzierenden Formkörper aus verpreßten Metalloxiden 40 bis 65 Gew.-% Kobalt, 10 bis 20 Gew.-% Mangan, 0,1 bis 0,5 Gew.-% Kupfer, 0,2 bis 5 Gew.-% Erdalkalimetall und gegebenenfalls 0,2 bis 5 Gew.-% Elemente der V. und/oder VI. Nebengruppe des Periodensystems, jeweils gerechnet als Metall, enthalten wobei sich die Prozentangaben auf die Gesamtmenge Metalloxid-Pulvergemisch beziehen und der Rest zu 100 Gew.-% Sauerstoff ist.

3. Verfahren nach Anspruch 1, dadurch gekennzeichnet, daß die Formkörper eine Druckfestigkeit von 300 bis 800 N/cm² bevorzugt 400 bis 600 N/cm², auf ebene Preßoberflächen, eine Druckfestigkeit von 50 bis 200 N, bevorzugt 80 bis 140 N, auf gewölbte Preßoberflächen und eine innere Oberfläche von 30 bis 200 m²/g , bevorzug 80 bis 160 m²/g, aufweisen.

4. Verfahren nach Anspruch 1, dadurch gekennzeichnet, daß bei einem H₂-Druck von 20 bis 400 bar, bevorzugt 100 bis 400 bar, besonders bevorzugt 200 bis 400 bar gearbeitet wird.

5. Verfahren nach Anspruch 1, dadurch gekennzeichnet, daß bei einer Temperatur von 160 bis 250°C gearbeitet wird.

6. Verfahren nach Anspruch 1, dadurch gekennzeichnet, daß kontinuierlich in der Gas- oder Rieselphase an fest angeordneten Katalysatoren gearbeitet wird und eine Katalysatorbelastung von 0,05 bis 2 kg, bevorzugt 0,1 bis 1 kg, besonders bevorzugt 0,15 bis 0,6 kg Diamino-toluole pro Liter Katalysator pro Stunde eingestellt wird.

7. Verfahren nach Anspruch 1, dadurch gekennzeichnet, daß die Formkörper vor ihrem Einsatz, bevorzugt nach Anordnung im Hydrierreaktor, durch Behandlung mit Wasserstoff bei einer Temperatur von 160 bis 280°C und bei 20 bis 300 bar reduziert werden, wobei der Wasserstoff zu Beginn der Reduktion als H₂/Inertgas-Gemisch einesetzt wird und der Inertgasanteil im Verlauf der Reduktion vollständig entfernt wird.

## Claims

1. Continuous process for preparing a mixture of amino-methyl-cyclohexanes and diamino-methyl-cyclohexanes of the formulae 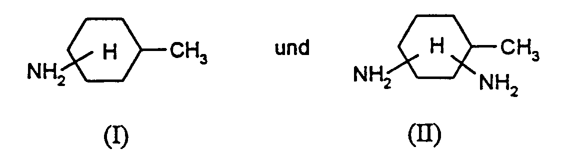 by catalytic hydrogenation of diamino-toluenes of the formula 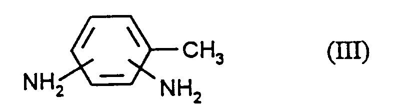 with hydrogen at reaction temperatures of from 150 to 260°C and an H₂ pressure of from 20 to 500 bar, characterized in that the catalysts used are support-free, reduced shaped bodies comprising pressed powders of cobalt, manganese, copper and alkaline earth metal (hydr)oxides, where the shaped bodies can have a content of one or more (hydr)oxides of metals of transition group V and/or VI of the Periodic Table of the Elements (Mendeleev).

2. Process according to Claim 1, characterized in that the shaped bodies comprising pressed metal oxides comprise, before reduction, from 40 to 65% by weight of cobalt, from 10 to 20% by weight of manganese, from 0.1 to 0.5% by weight of copper, from 0.2 to 5% by weight of alkaline earth metal and, if desired, from 0.2 to 5% by weight of elements of transition group V and/or VI of the Periodic Table, in each case calculated as metal, where the percentages are based on the total amount of metal oxide powder mixture and the remainder to 100% by weight is oxygen.

3. Process according to Claim 1, characterized in that the shaped bodies have a compressive strength of from 300 to 800 N/cm², preferably from 400 to 600 N/cm², on flat surfaces, a compressive strength of from 50 to 200 N, preferably from 80 to 140 N, on curved surfaces and an internal surface area of from 30 to 200 m²/g, preferably from 80 to 160 m²/g.

4. Process according to Claim 1, characterized in that it is carried out at an H₂ pressure of from 20 to 400 bar, preferably from 100 to 400 bar, particularly preferably from 200 to 400 bar.

5. Process according to Claim 1, characterized in that it is carried out at a temperature of from 160 to 250°C.

6. Process according to Claim 1, characterized in that it is carried out continuously in the gas phase or a trickling phase over fixed-bed catalysts at a weight hourly space velocity over the catalyst of from 0.05 to 2 kg, preferably from 0.1 to 1 kg, particularly preferably from 0.15 to 0.6 kg, of diamino-toluenes per litre of catalyst per hour.

7. Process according to Claim 1, characterized in that the shaped bodies are reduced by treatment with hydrogen at a temperature of from 160 to 280°C and at from 20 to 300 bar before use, preferably after installation in the hydrogenation reactor, where the hydrogen is used as an H₂/inert gas mixture at the beginning of the reduction and the proportion of inert gas is removed completely during the course of the reduction.

## Revendications

1. Procédé continu pour préparer un mélange d'aminométhylcyclohexanes et de diaminométhylcyclohexanes de formules : 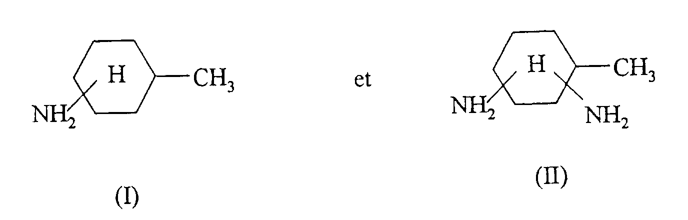 par hydrogénation catalytique de diaminotoluènes de formule 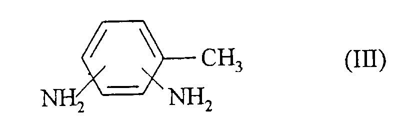 à des températures de réaction de 150 à 260°C sous une pression d'hydrogène de 20 à 500 bars, caractérisé en ce que l'on utilise en tant que catalyseurs des corps moulés par compression de poudres d'(hydr)oxydes de cobalt, de manganèse, de cuivre et d'un métal alcalino-terreux, ces corps moulés pouvant contenir un ou plusieurs (hydr)oxydes de métaux des sous-groupes V et/ou VI de la classification périodique des éléments (Mendeléieff), et étant exempts de matières de support, et réduits.

2. Procédé selon la revendication 1, selon lequel les corps moulés par compression d'oxydes métalliques à soumettre à réduction contiennent de 40 à 65% en poids de cobalt, de 10 à 20% en poids de manganèse, de 0,1 à 0,5 % en poids de cuivre, de 0,2 à 5 % en poids d'un métal alcalino-terreux et le cas échéant de 0,2 à 5 % en poids d'éléments des sous-groupes V et/ou VI de la classification périodique, dans tous les cas exprimé en métal, ces pourcentages se rapportant à la quantité totale du mélange pulvérulent d'oxydes métalliques et le complément à 100 % en poids consistant en oxygène.

3. Procédé selon la revendication 1, caractérisé en ce que les corps moulés ont une résistance à la compression de 300 à 800 N/cm², de préférence de 400 à 600 N/cm² sur la surface plane, une résistance à la compression de 50 à 200 N, de préférence de 80 à 140 N, sur les surfaces courbes, et une surface interne de 30 à 200 m²/g, de préférence de 80 à 160 m²/g.

4. Procédé selon la revendication 1, caractérisé en ce que l'on opère sous une pression d'hydrogène de 20 à 400 bars, de préférence de 100 à 400 bars et plus spécialement de 200 à 400 bars.

5. Procédé selon la revendication 1, caractérisé en ce que l'on opère à des températures de 160 et à 250°C.

6. Procédé selon la revendication 1 caractérisé en ce que l'on opère en continu en phase gazeuse ou en phase ruisselante sur le catalyseur en disposition fixe et on règle la charge du catalyseur à un niveau de 0,05 à 2 kg, de préférence de 0,1 à 1 kg et plus spécialement de 0,15 à 0,6 kg de diaminotoluènes par litre de catalyseur et par heure.

7. Procédé selon la revendication 1, caractérisé en ce que, avant leur utilisation et de préférence avant leur introduction dans le réacteur d'hydrogénation, les corps moulés sont réduits par traitement à l'hydrogène à des températures de 160 à 280°C sous des pressions de 20 à 300 bars, l'hydrogène étant mis en oeuvre au début de la réduction sous forme d'un mélange H₂/gaz inerte, et le gaz inerte étant totalement éliminé dans le cours de la réduction.
